# EUROPÄISCHE PATENTSCHRIFT

(11) **EP 4 474 033 B1**
(45) Veröffentlichungstag und Bekanntmachung des Hinweises auf die Patenterteilung: **25.02.2026**
(21) Anmeldenummer: 23177951.3
(22) Anmeldetag: 07.06.2023
(51) Int. Cl.: B01D 5/00, B01J 8/00, C07C 31/04, C07C 29/151, B01D 19/00

(54) **SYNTHESE VON METHANOL**
SYNTHESIS OF METHANOL
SYNTHÈSE DE MÉTHANOL

(43) Veröffentlichungstag der Anmeldung: 11.12.2024
(73) Patentinhaber: L'AIR LIQUIDE, SOCIETE ANONYME POUR L'ETUDE ET L'EXPLOITATION DES PROCEDES GEORGES CLAUDE, 75007 Paris (FR)
(72) Erfinder: Oelmann, Tobias, 60439 Frankfurt am Main (DE); Strozyk, Michael, 60439 Frankfurt am Main (DE); Peña, Vincent, 60439 Frankfurt am Main (DE)
(74) Vertreter: Air Liquide

(56) Entgegenhaltungen:
- WO-A1-2023/281238
- CN-U- 213 708 192

## Beschreibung

Die Erfindung betrifft ein Verfahren und eine Vorrichtung zur Synthese von Methanol.

Methanol kann mit einer Methanol-Anlage hergestellt werden, welche einen sogenannten Synthese-Loop aufweist. Dabei handelt es sich um ein Rohrleitungssystem, in welches typischerweise eine Rohgasquelle, ein Methanol-Synthesereaktor, Wärmeübertrager und ein Abscheider eingebunden sind.

Eines der Edukte der Methanol-Synthese ist Wasserstoff. Für eine möglichst umweltfreundliche Methanol-Synthese ist es wünschenswert, den Wasserstoff aus einer Wasserelektrolyse zu verwenden, welche mit erneuerbaren Energien betrieben wird. Erneuerbare Energien schwanken allerdings, so dass auch die Verfügbarkeit von Wasserstoff aus einer entsprechenden Quelle schwankt. Schwankungen im Feedgasstrom bewirken bei der Methanol-Synthese Druckschwankungen im Synthese-Loop. Ein schneller Druckabfall im Synthese-Loop kann allerdings zu Schäden an den verwendeten Installationen führen, insbesondere an einem bei der Methanol-Synthese verwendeten Katalysator. Daher ist es wünschenswert, den Druck im Synthese-Loop möglichst stabil zu halten. Die Dokumente WO 2023/281238 A1 und CN 213 708 192 u offenbaren Verfahren und Vorrichtungen zur Synthese von Methanol unter Verwendung eines Kreislaufsyntheseaufbaus.

Herkömmliche Methanol-Anlagen werden daher in der Regel nicht im Wechsellastbetrieb betrieben. Der Betrieb unter Teillast ist üblicherweise auf mindestens ca. 70% der Anlagenkapazitdat definiert. Der Teillastbetrieb wird bei einer herkömmlichen Methanol-Anlage üblicherweise nur bei reduzierter Gasverfügbarkeit wie beispielsweise im Winter gewählt, was über längere Zeiträume in der Größenordnung von Wochen und Monaten anhält und im Voraus planbar ist. Ein Wechsel in einen stationären Teillastbetrieb erfolgt somit über Stunden oder Tage mit sehr langsamen Prozessanpassungen zum Schutz der Equipments, insbesondere des Katalysators.

Besonders bei kleinen Anlagen, speziell z.B. bei CO₂-basierten Synthesen mit Wasserstoff aus einer Elektrolyse, ist ein Wechsellastbetrieb in einem Bereich zwischen 20 und 100 % wünschenswert, um beispielsweise der Verfügbarkeit von erneuerbarer Energie Rechnung zu tragen. Mit herkömmlichen Methanol-Anlagen kann dieser Wechsellastbetrieb nicht oder nur unter unverhältnismäßig großer Beanspruchung des Equipments realisiert werden.

Aufgabe der vorliegenden Erfindung ist es, eine Möglichkeit zu schaffen, Methanol auch bei schwankender Zufuhr der Edukte unter besonders geringer Beanspruchung des verwendeten Equipments herzustellen.

Diese Aufgaben werden gelöst mit dem Verfahren und der Vorrichtung gemäß den unabhängigen Ansprüchen. Weitere vorteilhafte Ausgestaltungen sind in den abhängigen Ansprüchen angegeben. Die in den Ansprüchen und in der Beschreibung dargestellten Merkmale sind in beliebiger, technologisch sinnvoller Weise miteinander kombinierbar.

Erfindungsgemäß wird ein Verfahren zur Synthese von Methanol unter Verwendung eines Rohrleitungssystems vorgestellt, in welches eine Rohgasquelle, ein Methanol-Synthesereaktor und eine Abscheideeinrichtung eingebunden sind, wobei ein Feedgasstrom umfassend Edukte für die Methanol-Synthese von der Rohgasquelle dem Methanol-Synthesereaktor mit einer Volumenströmungsrate zugeführt wird, wobei in dem Methanol-Synthesereaktor mit den Edukten Methanol erzeugt wird, wobei das erzeugte Methanol mit der Abscheideeinrichtung aus einem Auslassstrom des Methanol-Synthesereaktor abgeschieden wird, wobei weiterhin ein Hilfsvolumen derart in das Rohrleitungssystem eingebunden ist, dass ein für Gas zugängliches Volumen des Rohrleitungssystems von einem Füllstand einer Flüssigkeit in dem Hilfsvolumen abhängt, und wobei der Füllstand der Flüssigkeit in dem Hilfsvolumen zumindest in einem Falle einer Verringerung der Volumenströmungsrate des Feedgasstroms vergrößert wird.

Mit dem beschriebenen Verfahren kann Methanol unter Verwendung eines Rohrleitungssystems erzeugt werden. Das Rohrleitungssystem ist Teil einer Anordnung zur Synthese des Methanols. In das Rohrleitungssystem sind zumindest eine Rohgasquelle, ein Methanol-Synthesereaktor und eine Abscheideeinrichtung eingebunden. In das Rohrleitungssystem können auch weitere Komponenten eingebunden sein. Diese können wie bei einer herkömmlichen Anordnung für die Synthese von Methanol ausgebildet sein. Soweit solche weiteren Komponenten hierin nicht beschrieben sind, kommt es auf diese jedenfalls hinsichtlich der Besonderheiten des beschriebenen Verfahrens nicht an.

Auch kommt es nicht darauf an, wie das Rohrleitungssystem ausgebildet ist. Im einfachsten Fall umfasst das Rohrleitungssystem eine erste Leitung, über welche ein Auslass der Rohgasquelle mit einem Einlass des Methanol-Synthesereaktors verbunden ist sowie eine zweite Leitung, über welche ein Auslass des Methanol-Synthesereaktors mit einem Einlass der Abscheideeinrichtung verbunden ist. Vorzugsweise umfasst das Rohrleitungssystem weiterhin eine dritte Leitung, über welche ein Gasauslass der Abscheideeinrichtung mit der ersten Leitung verbunden ist. Über die dritte Leitung kann Gas in dem Rohrleitungssystem recycelt werden, also dem Methanol-Synthesereaktor erneut zugeführt werden. Sofern das Rohrleitungssystem die dritte Leitung oder eine äquivalente Möglichkeit zur Rezirkulierung aufweist, kann das Rohrleitungssystem auch als ein Loop bezeichnet werden, insbesondere als ein Synthese-Loop. Neben den beschriebenen zwei beziehungsweise drei Leitungen kann das Rohrleitungssystem verschiedene weitere Leitungen, Abzweige und Querverbindungen aufweisen.

Die Rohgasquelle kann eine Quelle für ein Rohgas sein, welches bereits ein Gemisch aller Edukte der Methanol-Synthese umfasst. Die Rohgasquelle kann aber auch mehrere Einzelquellen umfassen, welche jeweils nur eines der Edukte abgeben. Die entsprechenden Einzelströme können zu dem Feedgasstrom zusammengeführt werden. Wo dies erfolgt, ist für das beschriebene Verfahren unerheblich.

Bei dem beschriebenen Verfahren wird ein Feedgasstrom umfassend Edukte für die Methanol-Synthese von der Rohgasquelle dem Methanol-Synthesereaktor zugeführt. Dies erfolgt mit einer Volumenströmungsrate, auf welche unten näher eingegangen wird. Die Edukte für die Methanol-Synthese können insbesondere Wasserstoff einerseits sowie Kohlenmonoxid und/oder Kohlendioxid andererseits sein. Der Wasserstoff stammt vorzugsweise zumindest teilweise aus einer Wasserelektrolyse, welche mit erneuerbaren Energien betrieben wird.

In dem Methanol-Synthesereaktor wird mit den Edukten Methanol erzeugt. Dies kann auf eine für sich genommen bekannte Weise erfolgen, insbesondere unter Verwendung eines Katalysators in dem Methanol-Synthesereaktor. Aus dem Methanol-Synthesereaktor kann ein Auslassstrom austreten, welcher neben dem Methanol weitere Substanzen enthält, insbesondere bei der Methanol-Synthese gebildetes H₂O sowie die Edukte, soweit diese in dem Methanol-Synthesereaktor nicht reagiert sind. Das erzeugte Methanol wird mit der Abscheideeinrichtung aus dem Auslassstrom des Methanol-Synthesereaktors abgeschieden. Auch dies kann auf für sich genommen bekannte Weise erfolgen. Die Abscheideeinrichtung kann eine Flüssigkeits-Abscheideeinrichtung sein. Die Abscheideeinrichtung kann insbesondere zur Separierung von Gas- und Flüssigphase eingerichtet sein. In dem Fall kann das Methanol als Flüssigkeit abgeschieden werden. Für den Auslassstrom ist auch die Bezeichnung "Rohmethanol" gebräuchlich, obwohl der Auslassstrom neben dem Methanol auch andere Substanzen enthalten kann.

Im Unterschied zu herkömmlichen Verfahren zur Methanol-Synthese wird bei dem beschriebenen Verfahren das Volumen des Rohrleitungssystems gezielt verändert. Dabei wird der allgemeine Grundsatz ausgenutzt, dass bei einem Gas bei ansonsten unveränderten Bedingungen eine Reduzierung des verfügbaren Volumens mit einer Druckerhöhung einhergeht, und umgekehrt. Durch Veränderung des Volumens des Rohrleitungssystems kann also der Druck im Rohrleitungssystem beeinflusst werden. Das wird bei dem beschriebenen Verfahren ausgenutzt, um auf Schwankungen des Rohgasstroms reagieren zu können. Dies ist insbesondere dann vorteilhaft, wenn das Rohgas zumindest teilweise mit erneuerbaren Energien erzeugt wird, wie dies insbesondere bei Wasserstoff als Edukt für die Methanol-Synthese der Fall sein kann. Ist die erneuerbare Energie nicht ausreichend verfügbar, kann nicht ausreichend Wasserstoff für einen Volllastbetrieb der Methanol-Synthese hergestellt werden. Der Feedgasstrom wird mit einer verringerten Volumenströmungsrate dem Methanol-Synthesereaktor zugeführt. Dies würde grundsätzlich zu einer Verringerung des Drucks im Rohrleitungssystem führen, was wiederum zu Schäden an Komponenten führen kann, insbesondere am Katalysator.

Mit dem beschriebenen Verfahren kann eine solche Druckreduzierung verringert oder sogar ganz verhindert werden. Dazu wird zumindest in einem Falle einer Verringerung der Volumenströmungsrate des Feedgasstroms das für Gas zugängliche Volumen des Rohrleitungssystems verringert. Das führt dazu, dass trotzt der Verringerung der Volumenströmungsrate des Feedgasstroms der Druck im Rohrleitungssystem konstant gehalten wird oder nur wenig einbricht.

Das für Gas zugängliche Volumen des Rohrleitungssystems wird unter Verwendung eines Hilfsvolumens verändert. Das Hilfsvolumen ist derart in das Rohrleitungssystem eingebunden, dass das für Gas zugängliche Volumen des Rohrleitungssystems von einem Füllstand einer Flüssigkeit in dem Hilfsvolumen abhängt. Das Hilfsvolumen ist Teil des Rohrleitungssystems. Es kann in einer Leitung oder in einer in das Rohrleitungssystem eingebundenen Komponente ausgebildet sein. Das Hilfsvolumen ist ein Raum innerhalb des Rohrleitungssystems, welcher nicht zu allen Seiten geschlossen ist und insoweit mit dem übrigen Rohrleitungssystem fluidtechnisch verbunden ist. Es ist nicht erforderlich, dass das Hilfsvolumen durch ein gesondertes Bauteil gebildet ist oder anderweitig als solches vom übrigen Rohrleitungssystem zu unterscheiden ist. Es ist möglich, dass bei einem konkreten Rohrleitungssystem ein Hilfsvolumen auf mehrere verschiedene Arten und/oder an mehreren verschiedenen Orten identifiziert werden kann. Es genügt, dass sich in dem Hilfsvolumen Flüssigkeit sammeln kann. Das Hilfsvolumen kann beispielsweise in einer Senke einer Rohrleitung gebildet sein. Für die Funktionsweise des Verfahrens kommt es nicht auf die genaue Ausgestaltung des Hilfsvolumens an. Vielmehr werden die hierin beschriebenen Vorteile dadurch erreicht, dass das Hilfsvolumen wie beschrieben verwendet wird, um über den Füllstand der Flüssigkeit in dem Hilfsvolumen das für Gas zugängliche Volumen des Rohrleitungssystems einzustellen.

Das für Gas zugängliche Volumen des Rohrleitungssystems setzt sich insbesondere aus den Volumina der Leitungen des Rohrleitungssystems sowie aus Volumina der in das Rohrleitungssystem eingebundenen Komponenten zusammen. Beispielsweise ein für Gas zugänglicher Teil des Methanol-Synthesereaktors trägt zum für Gas zugänglichen Volumen des Rohrleitungssystems bei. Insoweit kann es dahinstehen, wo eine Grenze zwischen Methanol-Synthesereaktor und einer daran angebundenen Leitung gezogen wird.

Soweit das Hilfsvolumen nicht mit der Flüssigkeit gefüllt ist, trägt das Hilfsvolumen ebenfalls zum für Gas zugänglichen Volumen des Rohrleitungssystems bei. Der Teil des Hilfsvolumens, welcher mit der Flüssigkeit gefüllt ist, ist aufgrund der Flüssigkeit nicht für Gas zugänglich. Der für Gas zugängliche Anteil des Hilfsvolumens kann also über die Menge des Flüssigkeit in dem Hilfsvolumen variiert werden. So ist es möglich, das für Gas zugängliche Volumen des Rohrleitungssystems über einen Füllstand der Flüssigkeit in dem Hilfsvolumen zu beeinflussen. Bei dem beschriebenen Verfahren wird daher der Füllstand der Flüssigkeit in dem Hilfsvolumen zumindest in einem Falle einer Verringerung der Volumenströmungsrate des Feedgasstroms vergrößert. Durch Veränderung des Füllstandes der Flüssigkeit in dem Hilfsvolumen kann also auf einen Abfall der Volumenströmungsrate des Feedgasstroms reagiert werden.

Das beschriebene Verfahren ist vor allem dazu geeignet, auf schnelle Schwankungen im Feedgasstrom zu reagieren. Auf langsamere Schwankungen kann auch auf andere Weise reagiert werden. Um gegenüber einem herkömmlichen Verfahren einen Vorteil zu erzielen, genügt es daher, dass der Füllstand der Flüssigkeit in dem Hilfsvolumen zumindest in einem Falle einer Verringerung der Volumenströmungsrate des Feedgasstroms vergrößert wird. Auf das Verhalten des Füllstandes bei einer Vergrößerung der Volumenströmungsrate des Feedgasstroms kommt es im Allgemeinen nicht an. Auch ist der Füllstand der Flüssigkeit in dem Hilfsvolumen bei konstantem Feedgasstrom unbeachtlich. Es genügt bereits, dass der Füllstand zumindest in einem Falle einer Verringerung der Volumenströmungsrate des Feedgasstroms kurzzeitig vergrößert wird, um die Auswirkungen der Verringerung der Volumenströmungsrate des Feedgasstroms kurzfristig auszugleichen. Das liegt daran, dass der Druck in dem Rohrleitungssystem neben der Füllstandsregelung auch auf andere Arten beeinflusst werden kann. Hierzu kommen insbesondere alle Möglichkeiten in Betracht, welche auch bei einer herkömmlichen Anordnung zur Synthese von Methanol eingesetzt werden. Beispielsweise kann ein zu großer Druck über ein Überdruckventil abgelassen werden, welches vergleichsweise schnell reagieren kann. Im Allgemeinen kann zudem davon ausgegangen werden, dass es beispielsweise bei der Herstellung von Wasserstoff mittels Elektrolyse eher zu einem schnellen Abfall der Produktion kommt als zu einem schnellen Anstieg der Produktion. Die Gefahr eines zu großen Drucks unterscheidet sich also qualitativ von der Gefahr eines zu geringen Drucks.

Nichtsdestotrotz ist es bevorzugt, dass der Füllstand zumindest in einem Falle einer Vergrößerung der Volumenströmungsrate des Feedgasstroms verringert wird. Die Veränderung des Füllstandes trägt in dem Fall dazu bei, den Druck im Rohrleitungssystem zu stabilisieren. Vor dem Hintergrund des zuvor Gesagten ist es aber alternativ auch möglich, das der Füllstand der Flüssigkeit in dem Hilfsvolumen zunächst in einem Falle einer Verringerung der Volumenströmungsrate des Feedgasstroms vergrößert wird und anschließend unabhängig von der Volumenströmungsrate des Feedgasstroms wieder verringert wird, beispielsweise auf einen vor der Vergrößerung vorliegenden Wert. Wann diese Verringerung erfolgt, ist unerheblich.

Auch ist es nicht erforderlich, dass der Füllstand der Flüssigkeit in dem Hilfsvolumen jedes Mal vergrößert wird, wenn sich die Volumenströmungsrate des Feedgasstroms verringert. Es genügt, dass der Füllstand der Flüssigkeit in dem Hilfsvolumen zumindest in einem Falle einer Verringerung der Volumenströmungsrate des Feedgasstroms vergrößert wird, also mindestens einmal bei der Durchführung des Verfahrens. Insbesondere im Falle einer langsamen Verringerung der Volumenströmungsrate des Feedgasstroms ist es nicht erforderlich, mit einer Vergrößerung des Füllstandes der Flüssigkeit in dem Hilfsvolumen zu reagieren. Es ist jedoch bevorzugt, dass der Füllstand der Flüssigkeit in dem Hilfsvolumen jedes Mal vergrößert wird, wenn sich die Volumenströmungsrate des Feedgasstroms schneller als ein Druckveränderungs-Grenzwert verringert und/oder jedes Mal vergrößert wird, wenn sich die Volumenströmungsrate des Feedgasstroms auf einen Wert unterhalb eines Druck-Grenzwert verringert.

Die Flüssigkeit in dem Hilfsvolumen kann aufgrund ihrer Funktion auch als Hilfsflüssigkeit bezeichnet werden. Allerdings ist es nicht erforderlich, dass die Hilfsflüssigkeit nur für diesen Zweck verwendet wird. Die Hilfsflüssigkeit kann insbesondere auch eine ohnehin in dem Rohrleitungssystem vorhandene Flüssigkeit sein. Durch Vermeidung des Begriffs Hilfsflüssigkeit soll klargestellt werden, dass die Hilfsflüssigkeit keine gesonderte Flüssigkeit sein muss. Das Hilfsvolumen kann auch als ein Puffervolumen bezeichnet werden. Die Flüssigkeit in dem Hilfsvolumen kann entsprechend auch als eine Pufferflüssigkeit bezeichnet werden.

In einer bevorzugten Ausführungsform des Verfahrens umfasst die Flüssigkeit Methanol.

Die Verwendung von Methanol als die Flüssigkeit hat den Vorteil, dass keine Fremdsubstanz in das Rohrleitungssystem eingetragen werden muss. Vorzugsweise ist die Flüssigkeit reines Methanol. Das schließt nicht aus, dass das Methanol unvermeidliche Unreinheiten aufweist. Alternativ bevorzugt ist die Flüssigkeit ein auch als "Rohmethanol" oder "stabilisiertes Methanol" bezeichnetes Methanol-Wasser-Gemisch mit kleinen Mengen an Verunreinigungen, wie es bei der Methanolsynthese produziert wird.

Alternativ oder zusätzlich zu Methanol kann die Flüssigkeit aber auch andere Substanzen enthalten. Für die wesentliche Funktionsweise des beschriebenen Verfahrens kommt es lediglich darauf an, dass die Flüssigkeit Gas verdrängt und insoweit das für Gas zugängliche Volumen des Rohrleitungssystems beeinflussen kann. Dies ist allerdings bei Flüssigkeiten im Allgemeinen der Fall.

In einer weiteren bevorzugten Ausführungsform des Verfahrens umfasst die Flüssigkeit Methanol, welches im Methanol-Synthesereaktor erzeugt wurde. Dass die Flüssigkeit Methanol umfasst, bedeutet, dass die Flüssigkeit nicht zwingend reines Methanol ist, sondern insbesondere auch ein Gemisch aus Methanol und beispielsweise Wasser sein kann, welches auch unvermeidbare Nebenprodukte enthalten kann.

Die Verwendung von Methanol aus dem Methanol-Synthesereaktor als die Flüssigkeit hat den Vorteil, dass keine Flüssigkeit extern zugeführt werden muss. Das für Gas zugängliche Volumen des Rohrleitungssystems kann damit beeinflusst werden, indem lediglich der Füllstand des ohnehin im Rohrleitungssystem enthaltenen Methanols im Hilfsvolumen verändert wird. Dies ist beispielsweise über eine Pumpe möglich. Ein externer Speicher für die Flüssigkeit ist in dem Fall nicht erforderlich.

In einer weiteren bevorzugten Ausführungsform des Verfahrens wird der Füllstand der Flüssigkeit in dem Hilfsvolumen zumindest in einem Falle einer Verringerung der Volumenströmungsrate des Feedgasstroms vergrößert, indem die Flüssigkeit zumindest teilweise aus einem externen Speicher in das Hilfsvolumen eingeleitet wird.

Im Unterschied zu der zuvor beschriebenen Ausführungsform wird in dieser Ausführungsform ein externer Speicher verwendet. Der externe Speicher ist vorzugsweise ein Tank. Die Flüssigkeit im externen Speicher kann durch Methanol und/oder eine oder mehrere andere Substanzen gebildet sein. Die Flüssigkeit in dem externen Speicher kann insbesondere Rohmethanol, stabilisiertes Methanol, off-spec Methanol oder Reinmethanol sein.

Die Flüssigkeit im Hilfsvolumen kann durch Flüssigkeit aus dem externen Speicher und/oder durch andere Flüssigkeit, insbesondere Methanol aus dem Methanol-Synthesereaktor, gebildet sein. Denkbar ist also auch die Kombination der Ausführungsformen, wonach die Flüssigkeit Methanol umfasst, welches im Methanol-Synthesereaktor erzeugt wurde und der Füllstand der Flüssigkeit in dem Hilfsvolumen zumindest in einem Falle einer Verringerung der Volumenströmungsrate des Feedgasstroms vergrößert wird, indem die Flüssigkeit teilweise aus einem externen Speicher in das Hilfsvolumen eingeleitet wird. Im Falle dieser Kombination ist besonders bevorzugt, dass die Flüssigkeit in dem externen Speicher Methanol ist.

In einer weiteren bevorzugten Ausführungsform des Verfahrens wird mit dem Methanol-Synthesereaktor Methanol mit einer Erzeugungsrate von bis zu 5.000 Tonnen pro Tag erzeugt.

Je größer das Hilfsvolumen im Vergleich zum für Gas zugänglichen Volumen des übrigen Rohrleitungssystems ist, umso mehr kann das insgesamt für Gas zugängliche Volumen des Rohrleitungssystems über eine Füllstandsänderung der Flüssigkeit im Hilfsvolumen beeinflusst werden. Je größer diese Möglichkeit der Beeinflussung ist, umso mehr kann mit dem beschriebenen Verfahren auf eine Schwankung im Feedgasstrom reagiert werden und umso größer sind die Vorteile des beschriebenen Verfahrens. Es ist also erstrebenswert, dass das Hilfsvolumen im Vergleich zum für Gas zugänglichen Volumen des übrigen Rohrleitungssystems möglichst groß ist.

Aus praktischen Gründen ist die Realisierung des Hilfsvolumens im Allgemeinen umso schwieriger, je größer das Hilfsvolumen sein soll. Daher eignet sich das beschriebene Verfahren besonders für kleine bis mittelgroße Anordnungen zur Methanol-Synthese. In der vorliegenden Ausführungsform ist dies über die Erzeugungsrate des Methanols quantifiziert. Wird mit dem Methanol-Synthesereaktor Methanol mit einer Erzeugungsrate von beispielsweise 1.000 Tonnen pro Tag erzeugt, kann die Anordnung als eine mittelgroße Anordnung bezeichnet werden.

Für sehr kleine Anordnungen mit einer Erzeugungsrate von bis zu 10 Tonnen pro Tag kann das Hilfsvolumen unter verhältnismäßig geringem Kostenaufwand und Platzbedarf ein Mehrfaches des für Gas zugänglichen Volumens des übrigen Rohrleitungssystems sein. Somit können sehr kleine Anordnungen mithilfe des beschriebenen Verfahrens besonders flexibel betrieben werden.

Für kleine Anordnungen mit einer Erzeugungsrate zwischen 100 und 1.000 Tonnen pro Tag kann das Hilfsvolumen mit vertretbarem Aufwand dem für Gas zugänglichen Volumen des übrigen Rohrleitungssystems entsprechen. Somit kann das beschriebene Verfahren bei kleinen Anordnungen sinnvoll mit dem Ziel eines besonders flexiblen Betriebs eingesetzt werden.

Für mittelgroße Anlagen mit einer Erzeugungsrate zwischen 1.000 und 5.000 Tonnen pro Tag ist ein Hilfsvolumen in der Größenordnung des für Gas zugänglichen Volumens des übrigen Rohrleitungssystems möglich, stellt jedoch bereits einen relevanten Platzbedarf dar, sodass die Implementierung der vorgestellten Maßnahme hier mit zusätzlichen Herausforderungen verbunden sein kann.

Alternativ zu der beschriebenen Ausführungsform kann das Verfahren auch bei größeren Anlagen eingesetzt werden, indem der womöglich größere Aufwand zur Realisierung des Hilfsvolumens in Kauf genommen wird.

Als ein weiterer Aspekt der Erfindung wird eine Anordnung zur Synthese von Methanol vorgestellt. Die Anordnung umfasst ein Rohrleitungssystem sowie eine Rohgasquelle, einen Methanol-Synthesereaktor und eine Abscheideeinrichtung, welche in das Rohrleitungssystem eingebunden sind, wobei die Rohgasquelle, der Methanol-Synthesereaktor und die Abscheideeinrichtung über das Rohrleitungssystem miteinander verbunden sind, wobei die Anordnung weiterhin ein Hilfsvolumen umfasst, welches derart in das Rohrleitungssystem eingebunden ist, dass ein für Gas zugängliches Volumen des Rohrleitungssystems von einem Füllstand einer Flüssigkeit in dem Hilfsvolumen abhängt, und wobei die Anordnung weiterhin eine Füllstandsregelung umfasst, welche dazu eingerichtet ist, den Füllstand der Flüssigkeit in dem Hilfsvolumen zumindest in einem Falle einer Verringerung einer Volumenströmungsrate eines von der Rohgasquelle über das Rohrleitungssystem dem Methanol-Synthesereaktor zugeführten Feedgasstroms zu vergrößern.

Die Vorteile und Merkmale des Verfahrens sind auf die Anordnung anwendbar und übertragbar, und umgekehrt. Die Anordnung ist vorzugsweise zum Betrieb gemäß dem Verfahren eingerichtet. Das Verfahren wird vorzugsweise mit der Anordnung durchgeführt.

In das Rohrleitungssystem sind wie zum Verfahren beschrieben eine Rohgasquelle, ein Methanol-Synthesereaktor und eine Abscheideeinrichtung eingebunden. Die Rohgasquelle, der Methanol-Synthesereaktor und die Abscheideeinrichtung sind über das Rohrleitungssystem miteinander verbunden. Dass die Anordnung zur Synthese von Methanol eingerichtet ist, impliziert, dass die genannten Elemente so über das Rohrleitungssystem miteinander verbunden sind, dass in dem Methanol-Synthesereaktor Methanol erzeugt werden kann.

Die Rohgasquelle ist also derart mit dem Methanol-Synthesereaktor verbunden, dass ein Feedgasstrom umfassend Edukte für die Methanol-Synthese von der Rohgasquelle dem Methanol-Synthesereaktor mit einer Volumenströmungsrate zuführbar ist. Der Methanol-Synthesereaktor ist zur Erzeugung von Methanol mit den Edukten eingerichtet. Der Methanol-Synthesereaktor ist derart mit der Abscheideeinrichtung verbunden, dass das erzeugte Methanol mit der Abscheideeinrichtung aus einem Auslassstrom des Methanol-Synthesereaktor abscheidbar ist.

Die Füllstandsregelung ist dazu eingerichtet, den Füllstand der Flüssigkeit in dem Hilfsvolumen wie für das Verfahren beschrieben einzustellen. Die Füllstandsregelung umfasst alle dafür erforderlichen Elemente. Beispielsweise kann die Füllstandsregelung eine Steuereinrichtung umfassen, welche über ein Eingangssignal eine Information über die Volumenströmungsrate des Feedgasstroms erhält und über ein Ausgangssignal eine Pumpe derart steuert, dass der Füllstand der Flüssigkeit in dem Hilfsvolumen wie beschrieben eingestellt wird. Das Eingangssignal kann von einem Strömungssensor stammen, welcher ebenfalls Teil der Füllstandsregelung ist. Alternativ oder zusätzlich kann die Füllstandsregelung auch beispielsweise an eine nebengeordnete Steuereinheit angebunden sein oder in eine übergeordnete Steuereinheit integriert sein, wobei die Information über die Volumenströmungsrate des Feedgasstroms in der nebengeordneten Steuereinheit beziehungsweise der übergeordneten Steuereinheit ohnehin verfügbar ist. Auch die Pumpe kann Teil der Füllstandsregelung sein. Alternativ kann die Pumpe auch für andere Zwecke verwendet werden. Die Pumpe hat vorzugsweise einen Austrittsdruck im Bereich von 50 bis 150 bar, insbesondere im Bereich von 90 bis 100 bar.

In einer bevorzugten Ausführungsform der Anordnung ist das Hilfsvolumen zusammen mit der Abscheideeinrichtung in einem Abscheider ausgebildet.

In einer herkömmlichen Anordnung zur Synthese von Methanol wird das Methanol mit einem Abscheider aus dem Auslassstrom des Methanol-Synthesereaktors abgeschieden. Dieser Abscheider ist als gesondertes Bauteil in das Rohrleitungssystem eingebunden. In der vorliegenden Ausführungsform des beschriebenen Verfahrens ist jedoch auch das Hilfsvolumen in dem Abscheider ausgebildet. Daher wird hierin der Begriff der Abscheideeinrichtung verwendet. Die Abscheideeinrichtung umfasst die Elemente, die der Abscheidung des Methanols aus dem Auslassstrom des Methanol-Synthesereaktors dienen. In einer herkömmlichen Anordnung zur Synthese von Methanol können die Begriffe Abscheideeinrichtung und Abscheider synonym verwendet werden. In der vorliegenden Ausführungsform des beschriebenen Verfahrens ist jedoch der Abscheider die übergeordnete Einheit, welche die Abscheideeinrichtung und das Hilfsvolumen umfasst.

Auch ein herkömmlich verwendeter Abscheider hat an seiner Unterseite einen Bereich, in welchem sich Flüssigkeit sammeln kann. Der in der vorliegenden Ausführungsform des beschriebenen Verfahrens verwendete Abscheider kann daher vom Aufbau her sogar einem herkömmlichen Abscheider entsprechen. Bei einer herkömmlichen Anordnung ist der Abscheider allerdings nicht mit der hierin beschriebenen Füllstandsregelung mit dem Zweck der Beeinflussung des Drucks im Rohrleitungssystems kombiniert. Im einfachsten Fall unterscheidet sich die beschriebene Anordnung von einer herkömmlichen Anordnung also lediglich durch die Ausgestaltung der Steuerung. Die hierin beschriebenen Vorteile können allerdings in besonderem Maße erreicht werden, wenn die verwendete Anordnung mit Blick auf die Füllstandsregelung modifiziert ist. Insbesondere können die hierin beschriebenen Vorteile besonders dann erreicht werden, wenn das Hilfsvolumen besonders groß ist. Bei einem herkömmlichen Abscheider gibt es keinen vergleichbaren Anlass, ein möglichst großes Hilfsvolumen im Abscheider vorzusehen.

In einer weiteren bevorzugten Ausführungsform der Anordnung ist eine Querschnittsfläche des Abscheiders im Bereich des Hilfsvolumens mindestens 50 % größer als, bevorzugt doppelt so groß wie im Bereich der Abscheideeinrichtung.

In dieser Ausführungsform ist der Abscheider mit Blick auf die Füllstandsregelung modifiziert. Dazu ist der für das Hilfsvolumen verwendete Bereich des Abscheiders besonders groß. Der Abscheider kann dadurch als bauchig bezeichnet werden.

Alternativ oder zusätzlich zu einem besonders großen Querschnitt im Beriech des Hilfsvolumens kann ein besonders großes Hilfsvolumen auch durch eine besonders große axiale Ausdehnung des Abscheiders erreicht werden. Der besonders große Querschnitt im Bereich des Hilfsvolumens hat jedoch den Vorteil, dass auch bei einer stehenden Anordnung des Abscheiders keine besonders große Pumpleistung erforderlich ist, um die Flüssigkeit in das Hilfsvolumen einzuleiten.

In einer weiteren bevorzugten Ausführungsform der Anordnung ist das Hilfsvolumen unterhalb der Abscheideeinrichtung angeordnet.

Auch in einem herkömmlichen Abscheider ist im Allgemeinen unterhalb der Abscheideeinrichtung ein Bereich vorgesehen, in welchem sich das abgeschiedene flüssige Methanol sammeln kann. Insoweit kommt die beschriebene Anordnung in der vorliegenden Ausführungsform ohne große Umbaumaßnahmen aus. Denkbar ist sogar, einen herkömmlichen Abscheider unverändert zu verwenden. Die hierin beschriebene Erkenntnis ist jedoch, den unteren Bereich des Abscheiders als das Hilfsvolumen zu verwenden und mit der beschriebenen Füllstandsregelung zu kombinieren.

Die Flüssigkeit kann insbesondere mit dem Methanol-Synthesereaktor erzeugtes Rohmethanol sein, welches in einem oberen Teil eines Abscheiders durch die Abscheideeinrichtung abgeschieden wurde und in das unterhalb der Abscheideeinrichtung angeordnete Hilfsvolumen abgeschieden wurde und nach unten in das Hilfsvolumen gelaufen oder getropft ist.

Das Hilfsvolumen unterhalb der Abscheideeinrichtung anzuordnen hat allgemein den Vorteil, dass die Abscheideeinrichtung dadurch stromabwärts des Hilfsvolumens angeordnet ist. Selbst wenn ein Teil der Flüssigkeit aus dem Hilfsvolumen durch einen Gasstrom im Rohrleitungssystem mitgenommen werden sollte, würde dieser Teil der Flüssigkeit sofort wieder durch die Abscheideeinrichtung aus dem Gasstrom abgeschieden. Es besteht also keine Gefahr, dass Tröpfchen mit dem Gasstrom mitwandern. Letzteres ist insbesondere für den bevorzugten Fall relevant, dass die Anordnung so ausgebildet ist, dass der Auslassstrom des Methanol-Synthesereaktors rezirkuliert werden kann. In dem Fall könnten die mitgenommenen Tröpfchen in eine Recycle-Leitung gelangen. Durch die Abscheideeinrichtung kann dies verhindert werden.

In einer weiteren bevorzugten Ausführungsform der Anordnung ist das Hilfsvolumen in einem Behälter ausgebildet, welcher zwischen dem Methanol-Synthesereaktor und der Abscheideeinrichtung in das Rohrleitungssystem eingebunden ist.

Alternativ zu den zuvor beschriebenen Ausführungsformen, bei denen das Hilfsvolumen in dem Abscheider ausgebildet ist, ist das Hilfsvolumen in dieser Ausführungsform außerhalb des Abscheiders ausgebildet.

In einer weiteren bevorzugten Ausführungsform der Anordnung ist der Behälter als eine Knock-Out-Drum ausgebildet.

In dieser Ausführungsform ist das Hilfsvolumen in eine erste Stufe der Abscheidung vor dem eigentlichen Abscheider integriert.

In einer weiteren bevorzugten Ausführungsform der Anordnung sind mindestens 10 %, vorzugsweise mindestens 30 % eines für Fluid zugänglichen Gesamtvolumens des Rohrleitungssystems durch das Hilfsvolumen gebildet.

Je größer das Hilfsvolumen im Vergleich zum für Gas zugänglichen Volumen des übrigen Rohrleitungssystems ist, umso mehr kann das insgesamt für Gas zugängliche Volumen des Rohrleitungssystems über eine Füllstandsänderung der Flüssigkeit im Hilfsvolumen beeinflusst werden. Daher ist bevorzugt, dass ein für Fluid zugängliches Gesamtvolumen des Rohrleitungssystems zu mindestens 10 %, insbesondere zu mindestens 30 % durch das Hilfsvolumen gebildet ist. Insbesondere durch diese quantitative Angabe unterscheidet sich die beschriebene Anordnung von einer herkömmlichen Anordnung, bei welcher es keinen Anlass für ein derart großes Hilfsvolumen gibt.

Ein Fluid kann ein Gas und/oder eine Flüssigkeit sein. Das für Fluid zugängliche Volumen des Rohrleitungssystems setzt sich also durch das für Gas zugängliche Volumen und das von Flüssigkeit eingenommene Volumen zusammen.

In einer weiteren bevorzugten Ausführungsform umfasst die Anordnung weiterhin einen zumindest teilweise mit der Flüssigkeit gefüllten externen Speicher, welcher an das Hilfsvolumen angebunden ist.

In der vorliegenden Ausführungsform kann der Füllstand der Flüssigkeit in dem Hilfsvolumen zumindest in einem Falle einer Verringerung der Volumenströmungsrate des Feedgasstroms vergrößert werden, indem die Flüssigkeit zumindest teilweise aus dem externen Speicher in das Hilfsvolumen eingeleitet wird. Die Anordnung ist also derart ausgebildet, dass Flüssigkeit von dem externen Speicher in das Hilfsvolumen einleitbar ist.

Der externe Speicher ist vorzugsweise über eine Leitung an das Hilfsvolumen angebunden, welche von einer Verbindungsleitung zwischen dem Methanol-Synthesereaktor zum Abscheider verschieden ist.

Insbesondere durch den externen Speicher unterscheidet sich die beschriebene Anordnung von einer herkömmlichen Anordnung, bei welcher es keinen Anlass für einen solchen externen Speicher gibt.

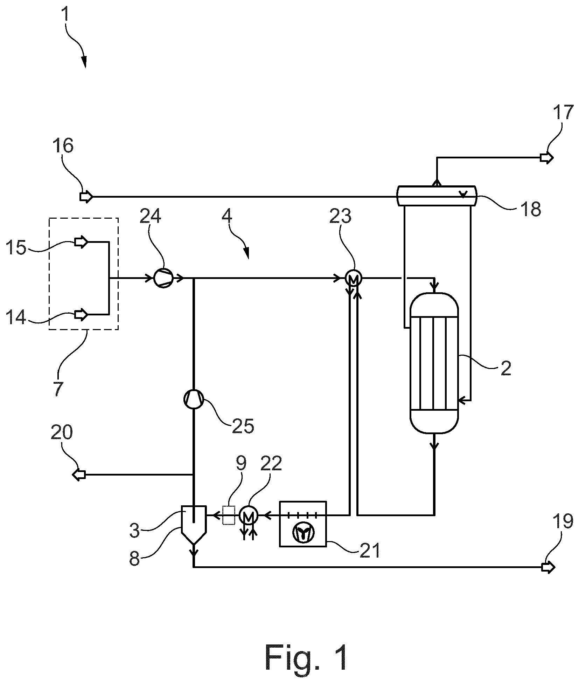

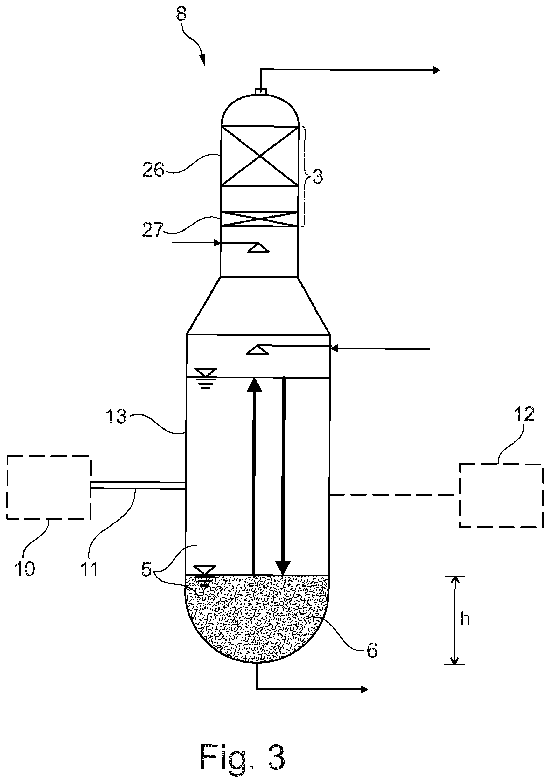

Die Erfindung wird nachfolgend anhand der Figuren näher erläutert. Die Figuren 1 und 3 zeigen ein besonders bevorzugtes Ausführungsbeispiel, auf das die Erfindung jedoch nicht begrenzt ist. Die Figuren und die darin dargestellten Größenverhältnisse sind nur schematisch. Es zeigen:
- Fig. 1:: eine erfindungsgemäße Anordnung zur Synthese von Methanol,
- Fig. 2:: einen aus dem Stand der Technik bekannten Abscheider, und
- Fig. 3:: den Abscheider der erfindungsgemäßen Anordnung aus Fig. 1.

Fig. 1 zeigt eine Anordnung 1 zur Synthese von Methanol. Die Anordnung 1 umfasst ein Rohrleitungssystem 4 sowie eine Rohgasquelle 7, einen Methanol-Synthesereaktor 2 und eine Abscheideeinrichtung 3, welche in das Rohrleitungssystem 4 eingebunden sind. Die Abscheideeinrichtung 3 ist in einem Abscheider 8 ausgebildet, was in Fig. 3 im Detail zu erkennen ist. Die Rohgasquelle 7 umfasst eine H₂-Quelle 14 und eine CO₂-Quelle 15. Die Rohgasquelle 7, der Methanol-Synthesereaktor 2 und die Abscheideeinrichtung 3 in dem Abscheider 8 sind über das Rohrleitungssystem 4 miteinander verbunden. Weiterhin umfasst die Anordnung 1 eine Wasserzuleitung 16, welche mit einer Dampftrommel 18 verbunden ist. Von der Dampftrommel 18 ausgehender Dampf kann über einen Dampfauslass 17 ausgelassen werden.

In das Rohrleitungssystem 4 sind weiterhin ein erster Kühler 21, ein zweiter Kühler 22, ein Wärmetauscher 23, ein erster Verdichter 24 und ein zweiter Verdichter 25 eingebunden. Optional kann zudem ein Behälter 9 zwischen dem Methanol-Synthesereaktor 2 und der Abscheideeinrichtung 3 in das Rohrleitungssystem 4 eingebunden sein. Dieser ist gestrichelt an einer beispielhaften Position eingezeichnet. Der Behälter 9 kann als eine Knock-Out-Drum ausgebildet sein.

Das mit dem Methanol-Synthesereaktor 2 gebildete Methanol kann mit der Abscheideeinrichtung 3 abgeschieden und an einem Methanolauslass 19 ausgelassen werden. Im Falle eines Überdrucks im Rohrleitungssystem 4 kann Gas über einen Überdruckablass 20 abgelassen werden.

Mit dem Methanol-Synthesereaktor 2 der Anordnung 1 kann Methanol mit einer Erzeugungsrate von bis zu als 5.000 Tonnen pro Tag erzeugt werden.

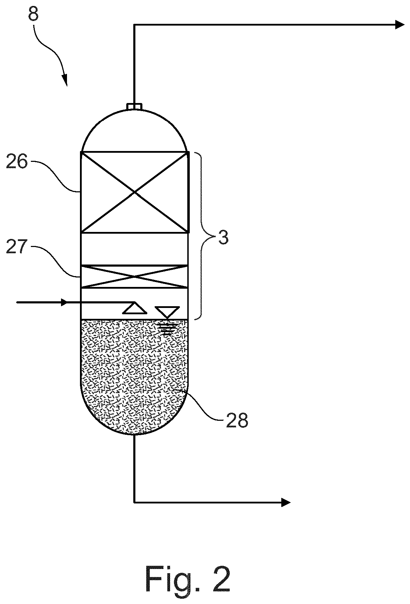

Fig. 2 zeigt einen aus dem Stand der Technik bekannten Abscheider 8. Dieser umfasst eine Abscheideeinrichtung 3, welche durch einen Hauptabscheider 26 und einen Vorabscheider 27 gebildet ist. Im unteren Bereich des Abscheiders 8 ist ein Flüssigkeitsbereich 28 vorgesehen.

Fig. 3 zeigt den Abscheider 8 der erfindungsgemäßen Anordnung 1 aus Fig. 1. Auch dieser umfasst eine Abscheideeinrichtung 3, welche durch einen Hauptabscheider 26 und einen Vorabscheider 27 gebildet ist.

Durch den Abscheider 8 aus Fig. 3 umfasst die Anordnung 1 ein Hilfsvolumen 5, welches derart in das Rohrleitungssystem 4 eingebunden ist, dass ein für Gas zugängliches Volumen des Rohrleitungssystems 4 von einem Füllstand h einer Flüssigkeit 6 in dem Hilfsvolumen 5 abhängt.

Die Anordnung 1 umfasst weiterhin eine Füllstandsregelung 12, welche dazu eingerichtet ist, den Füllstand h der Flüssigkeit 6 in dem Hilfsvolumen 5 zumindest in einem Falle einer Verringerung einer Volumenströmungsrate eines von der Rohgasquelle 7 über das Rohrleitungssystem 4 dem Methanol-Synthesereaktor 2 zugeführten Feedgasstroms zu vergrößern. Die Füllstandsregelung 12 ist der Übersichtlichkeit halber nur in Fig. 3, nicht jedoch in Fig. 1 gezeigt.

Die Abscheideeinrichtung 3 und das Hilfsvolumen 5 sind in einem gemeinsamen Gehäuse 13 ausgebildet. Das Hilfsvolumen 5 ist unterhalb der Abscheideeinrichtung 3 angeordnet.

Im Unterschied zum Abscheider 8 aus Fig. 2 ist der Abscheider 8 aus Fig. 3 im unteren Bereich bauchig ausgebildet. Ein Querschnitt des Abscheiders 8 aus Fig. 3 ist daher im Bereich des Hilfsvolumens 5 mehr als doppelt so groß wie im Bereich der Abscheideeinrichtung 3. Dadurch ist es möglich, dass mindestens 30 % eines für Fluid zugänglichen Gesamtvolumens des Rohrleitungssystems 4 durch das Hilfsvolumen 5 gebildet sind.

Weiterhin umfasst die Anordnung 1 einen zumindest teilweise mit der Flüssigkeit 6 gefüllten externen Speicher 10, welcher über eine Verbindungsleitung 11 an das Hilfsvolumen 5 angebunden ist. Dies ist Fig. 3 gezeigt, in Fig. 1 der Übersichtlichkeit halber jedoch nicht.

### Bezugszeichenliste

- 1: Anordnung
- 2: Methanol-Synthesereaktor
- 3: Abscheideeinrichtung
- 4: Rohrleitungssystem
- 5: Hilfsvolumen
- 6: Flüssigkeit
- 7: Rohgasquelle
- 8: Abscheider
- 9: Behälter
- 10: externer Speicher
- 11: Verbindungsleitung
- 12: Füllstandsregelung
- 13: Gehäuse
- 14: H₂-Quelle
- 15: CO₂-Quelle
- 16: Wasserzuleitung
- 17: Dampfauslass
- 18: Dampftrommel
- 19: Methanolauslass
- 20: Überdruckablass
- 21: erster Kühler
- 22: zweiter Kühler
- 23: Wärmetauscher
- 24: erster Verdichter
- 25: zweiter Verdichter
- 26: Hauptabscheider
- 27: Vorabscheider
- 28: Flüssigkeitsbereich
- h: Füllstand

## Patentansprüche

1. Verfahren zur Synthese von Methanol unter Verwendung eines Rohrleitungssystems (4), in welches eine Rohgasquelle (7), ein Methanol-Synthesereaktor (2) und eine Abscheideeinrichtung (3) eingebunden sind, wobei ein Feedgasstrom umfassend Edukte für die Methanol-Synthese von der Rohgasquelle (7) dem Methanol-Synthesereaktor (2) mit einer Volumenströmungsrate zugeführt wird, wobei in dem Methanol-Synthesereaktor (2) mit den Edukten Methanol erzeugt wird, wobei das erzeugte Methanol mit der Abscheideeinrichtung (3) aus einem Auslassstrom des Methanol-Synthesereaktor (2) abgeschieden wird, wobei weiterhin ein Hilfsvolumen (5) derart in das Rohrleitungssystem (4) eingebunden ist, dass ein für Gas zugängliches Volumen des Rohrleitungssystems (4) von einem Füllstand (h) einer Flüssigkeit (6) in dem Hilfsvolumen (5) abhängt, und wobei der Füllstand (h) der Flüssigkeit (6) in dem Hilfsvolumen (5) zumindest in einem Falle einer Verringerung der Volumenströmungsrate des Feedgasstroms vergrößert wird.

2. Verfahren nach Anspruch 1, wobei die Flüssigkeit (6) Methanol umfasst.

3. Verfahren nach Anspruch 2, wobei die Flüssigkeit (6) Methanol umfasst, welches im Methanol-Synthesereaktor (2) erzeugt wurde.

4. Verfahren nach einem der vorhergehenden Ansprüche, wobei der Füllstand (h) der Flüssigkeit (6) in dem Hilfsvolumen (5) zumindest in einem Falle einer Verringerung der Volumenströmungsrate des Feedgasstroms vergrößert wird, indem die Flüssigkeit (6) zumindest teilweise aus einem externen Speicher (10) in das Hilfsvolumen (5) eingeleitet wird.

5. Verfahren nach einem der vorhergehenden Ansprüche, wobei mit dem Methanol-Synthesereaktor (2) Methanol mit einer Erzeugungsrate von bis zu 5.000 Tonnen pro Tag erzeugt wird.

6. Anordnung (1) zur Synthese von Methanol, umfassend ein Rohrleitungssystem (4) sowie eine Rohgasquelle (7), einen Methanol-Synthesereaktor (2) und eine Abscheideeinrichtung (3), welche in das Rohrleitungssystem (4) eingebunden sind, wobei die Rohgasquelle (7), der Methanol-Synthesereaktor (2) und die Abscheideeinrichtung (3) über das Rohrleitungssystem (4) miteinander verbunden sind, wobei die Anordnung (1) weiterhin ein Hilfsvolumen (5) umfasst, welches derart in das Rohrleitungssystem (4) eingebunden ist, dass ein für Gas zugängliches Volumen des Rohrleitungssystems (4) von einem Füllstand (h) einer Flüssigkeit (6) in dem Hilfsvolumen (5) abhängt, und wobei die Anordnung (1) weiterhin eine Füllstandsregelung (12) umfasst, welche dazu eingerichtet ist, den Füllstand (h) der Flüssigkeit (6) in dem Hilfsvolumen (5) zumindest in einem Falle einer Verringerung einer Volumenströmungsrate eines von der Rohgasquelle (7) über das Rohrleitungssystem (4) dem Methanol-Synthesereaktor (2) zugeführten Feedgasstroms zu vergrößern.

7. Anordnung (1) nach Anspruch 6, wobei das Hilfsvolumen (5) zusammen mit der Abscheideeinrichtung (3) in einem Abscheider (8) ausgebildet ist.

8. Anordnung (1) nach Anspruch 7, wobei eine Querschnittsfläche des Abscheiders (8) im Bereich des Hilfsvolumens (5) mindestens 50 % größer ist als im Bereich der Abscheideeinrichtung (3).

9. Anordnung (1) nach einem der Ansprüche 6 bis 8, wobei das Hilfsvolumen (5) unterhalb der Abscheideeinrichtung (3) angeordnet ist.

10. Anordnung (1) nach Anspruch 6, wobei das Hilfsvolumen (5) in einem Behälter (9) ausgebildet ist, welcher zwischen dem Methanol-Synthesereaktor (2) und der Abscheideeinrichtung (3) in das Rohrleitungssystem (4) eingebunden ist.

11. Anordnung (1) nach Anspruch 10, wobei der Behälter (9) als eine Knock-Out-Drum ausgebildet ist.

12. Anordnung (1) nach einem der Ansprüche 6 bis 11, wobei mindestens 10 % eines für Fluid zugänglichen Gesamtvolumens des Rohrleitungssystems (4) durch das Hilfsvolumen (5) gebildet sind.

13. Anordnung (1) nach einem der Ansprüche 6 bis 12, weiterhin umfassend einen zumindest teilweise mit der Flüssigkeit (6) gefüllten externen Speicher (10), welcher an das Hilfsvolumen (5) angebunden ist.

## Claims

1. A method for synthesizing methanol using a piping system (4), into which a raw gas source (7), a methanol synthesis reactor (2), and a separation device (3) are integrated, wherein a feed gas stream comprising educts for the methanol synthesis is supplied from the raw gas source (7) to the methanol synthesis reactor (2) at a volumetric flow rate, wherein methanol is produced in the methanol synthesis reactor (2) with the educts, wherein the produced methanol is separated by the separation device (3) from an outlet stream of the methanol synthesis reactor (2), wherein furthermore an auxiliary volume (5) is integrated into the piping system (4) in such a way that a gas-accessible volume of the piping system (4) depends on a fill level (h) of a liquid (6) in the auxiliary volume (5), and wherein the fill level (h) of the liquid (6) in the auxiliary volume (5) is increased in at least one case of a reduction in the volumetric flow rate of the feed gas stream.

2. The method according to claim 1, wherein the liquid (6) comprises methanol.

3. The method according to claim 2, wherein the liquid (6) comprises methanol which was produced in the methanol synthesis reactor (2).

4. The method according to any one of the preceding claims, wherein the fill level (h) of the liquid (6) in the auxiliary volume (5) is increased in at least one case of a reduction in the volumetric flow rate of the feed gas stream by at least partially introducing the liquid (6) from an external storage (10) into the auxiliary volume (5).

5. The method according to any one of the preceding claims, wherein methanol is produced with the methanol synthesis reactor (2) at a production rate of up to 5,000 tons per day.

6. An arrangement (1) for synthesizing methanol, comprising a piping system (4) as well as a raw gas source (7), a methanol synthesis reactor (2), and a separation device (3), which are integrated into the piping system (4), wherein the raw gas source (7), the methanol synthesis reactor (2), and the separation device (3) are interconnected via the piping system (4), wherein the arrangement (1) further comprises an auxiliary volume (5), which is integrated into the piping system (4) in such a way that a gas-accessible volume of the piping system (4) depends on a fill level (h) of a liquid (6) in the auxiliary volume (5), and wherein the arrangement (1) further comprises a fill level control (12), which is configured to increase the fill level (h) of the liquid (6) in the auxiliary volume (5) in at least one case of a reduction in a volumetric flow rate of a feed gas stream supplied from the raw gas source (7) via the piping system (4) to the methanol synthesis reactor (2).

7. The arrangement (1) according to claim 6, wherein the auxiliary volume (5) is formed together with the separation device (3) in a separator (8).

8. The arrangement (1) according to claim 7, wherein a cross-sectional area of the separator (8) in the region of the auxiliary volume (5) is at least 50% larger than in the region of the separation device (3).

9. The arrangement (1) according to any one of claims 6 to 8, wherein the auxiliary volume (5) is arranged below the separation device (3).

10. The arrangement (1) according to claim 6, wherein the auxiliary volume (5) is formed in a container (9) which is integrated between the methanol synthesis reactor (2) and the separation device (3) into the piping system (4).

11. The arrangement (1) according to claim 10, wherein the container (9) is configured as a knock-out drum.

12. The arrangement (1) according to any one of claims 6 to 11, wherein at least 10% of a total fluid-accessible volume of the piping system (4) is formed by the auxiliary volume (5).

13. The arrangement (1) according to any one of claims 6 to 12, further comprising an external storage (10) at least partially filled with the liquid (6), which is connected to the auxiliary volume (5).

## Revendications

1. Procédé de synthèse de méthanol utilisant un système de tuyauterie (4), dans lequel sont intégrés une source de gaz brut (7), un réacteur de synthèse de méthanol (2) et un dispositif de séparation (3), dans lequel un flux de gaz d'alimentation comprenant des réactifs pour la synthèse de méthanol est amené de la source de gaz brut (7) au réacteur de synthèse de méthanol (2) avec un débit volumique, dans lequel du méthanol est produit dans le réacteur de synthèse de méthanol (2) avec les réactifs, dans lequel le méthanol produit est séparé par le dispositif de séparation (3) d'un flux de sortie du réacteur de synthèse de méthanol (2), dans lequel en outre un volume auxiliaire (5) est intégré dans le système de tuyauterie (4) de telle sorte qu'un volume accessible au gaz du système de tuyauterie (4) dépend d'un niveau de remplissage (h) d'un liquide (6) dans le volume auxiliaire (5), et dans lequel le niveau de remplissage (h) du liquide (6) dans le volume auxiliaire (5) est augmenté au moins dans un cas de diminution du débit volumique du flux de gaz d'alimentation.

2. Procédé selon la revendication 1, dans lequel le liquide (6) comprend du méthanol.

3. Procédé selon la revendication 2, dans lequel le liquide (6) comprend du méthanol, qui a été produit dans le réacteur de synthèse de méthanol (2).

4. Procédé selon l'une des revendications précédentes, dans lequel le niveau de remplissage (h) du liquide (6) dans le volume auxiliaire (5) est augmenté au moins dans un cas de diminution du débit volumique du flux de gaz d'alimentation, en introduisant le liquide (6) au moins partiellement depuis un réservoir externe (10) dans le volume auxiliaire (5).

5. Procédé selon l'une des revendications précédentes, dans lequel du méthanol est produit avec le réacteur de synthèse de méthanol (2) avec un taux de production allant jusqu'à 5 000 tonnes par jour.

6. Agencement (1) pour la synthèse de méthanol, comprenant un système de tuyauterie (4) ainsi qu'une source de gaz brut (7), un réacteur de synthèse de méthanol (2) et un dispositif de séparation (3), qui sont intégrés dans le système de tuyauterie (4), dans lequel la source de gaz brut (7), le réacteur de synthèse de méthanol (2) et le dispositif de séparation (3) sont reliés entre eux par le système de tuyauterie (4), dans lequel l'agencement (1) comprend en outre un volume auxiliaire (5), qui est intégré dans le système de tuyauterie (4) de telle sorte qu'un volume accessible au gaz du système de tuyauterie (4) dépend d'un niveau de remplissage (h) d'un liquide (6) dans le volume auxiliaire (5), et dans lequel l'agencement (1) comprend en outre une régulation de niveau de remplissage (12), qui est configurée pour augmenter le niveau de remplissage (h) du liquide (6) dans le volume auxiliaire (5) au moins dans un cas de diminution d'un débit volumique d'un flux de gaz d'alimentation amené de la source de gaz brut (7) par le système de tuyauterie (4) au réacteur de synthèse de méthanol (2).

7. Agencement (1) selon la revendication 6, dans lequel le volume auxiliaire (5) est formé conjointement avec le dispositif de séparation (3) dans un séparateur (8).

8. Agencement (1) selon la revendication 7, dans lequel une surface de section transversale du séparateur (8) dans la zone du volume auxiliaire (5) est au moins 50 % plus grande que dans la zone du dispositif de séparation (3).

9. Agencement (1) selon l'une des revendications 6 à 8, dans lequel le volume auxiliaire (5) est disposé en dessous du dispositif de séparation (3).

10. Agencement (1) selon la revendication 6, dans lequel le volume auxiliaire (5) est formé dans un récipient (9) qui est intégré entre le réacteur de synthèse de méthanol (2) et le dispositif de séparation (3) dans le système de tuyauterie (4).

11. Agencement (1) selon la revendication 10, dans lequel le récipient (9) est configuré comme un ballon de détente (knock-out drum).

12. Agencement (1) selon l'une des revendications 6 à 11, dans lequel au moins 10 % d'un volume total accessible au fluide du système de tuyauterie (4) est constitué par le volume auxiliaire (5).

13. Agencement (1) selon l'une des revendications 6 à 12, comprenant en outre un réservoir externe (10) rempli au moins partiellement avec le liquide (6), qui est raccordé au volume auxiliaire (5).
